# EUROPEAN PATENT SPECIFICATION

(11) **EP 3 888 660 B1**
(45) Date of publication and mention of the grant of the patent: **14.08.2024**
(21) Application number: 19888477.7
(22) Date of filing: 28.10.2019
(51) Int. Cl.: A61K 31/5575, A61P 9/08, A61P 9/00, A61P 9/10, A61P 9/04, A61P 7/02, A61P 3/10, A61P 17/00, A61P 17/10

(54) **USE OF PROSTAGLANDIN E1 METHYL ESTER FOR TREATING SCLERODERMA**
VERWENDUNG VON PROSTAGLANDIN E1-METHYLESTER ZUR BEHANDLUNG VON DERMATOSKLEROSE
UTILISATION DE MÉTHYL ESTER DE PROSTAGLANDINE E1 DANS LE TRAITEMENT DE LA SCLÉRODERMIE

(30) Priority: 27.11.2018 CN 201811423944
(43) Date of publication of application: 06.10.2021
(73) Proprietor: Xi'an Libang Zhaoxin Biotechnology Co., Ltd., Xi'an, Shaanxi 710069 (CN)
(72) Inventor: WANG, Rutao, Xi' an City, Shaanxi 710069 (CN); AN, Long, Xi' an City, Shaanxi 710069 (CN); ZHAO, Yi, Xi' an City, Shaanxi 710069 (CN); PANG, Jinghua, Xi' an City, Shaanxi 710069 (CN); CHEN, Tao, Xi' an City, Shaanxi 710069 (CN)
(74) Representative: Dehns
(86) International application number: PCT/CN2019/113558
(87) International publication number: WO 2020/108194

(56) References cited:
- WO-A1-2012/139033
- WO-A2-03/057162
- WO-A2-03/092617
- CN-A- 109 288 848
- CN-A- 109 394 704
- JP-A- H08 283 160
- US-A- 5 219 885
- LONGHUA WANG ZHENGYUAN: "Efficacy of prostaglandin E1 and sodium alginate in the treatment of scleroderma", CHIN J DERMATOL., vol. 29, no. 4, 31 August 1996 (1996-08-31), pages 284 - 285, XP055825453
- BAI, HUA ET AL.: "Clinical Application Status of Prostaglandin E1", CHINESE JOURNAL OF CLINICAL RATIONAL DRUG USE, vol. 8, no. 12A, 31 December 2015 (2015-12-31), pages 177 - 180, XP009528440, DOI: 10.15887/j.cnki.13-1389/r.2015.34.112

## Description

### Technical Field

The disclosure relates to the field of medicine. Specifically, the disclosure relates to prostaglandin E1 methyl ester for use in a method of treating scleroderma.

### Background of Art

Prostaglandin E1 (PGE1) is a natural endogenous vasodilator, which can be synthesized by human cells. It is an important substance that regulates cell function. It does not accumulate in the body, does not produce tolerance, and it's non-toxic without damaging side effects. It has a definite therapeutic effect and is superior to exogenous drugs. Prostaglandin E1 has extremely strong physiological activity and a wide range of pharmacological activities. It can be used clinically in cardiovascular and cerebrovascular diseases, diabetic complications, respiratory diseases, pulmonary hypertension, hepatorenal syndrome (HRS), liver failure, nephropathy, etc. Studies have found that prostaglandin E1 not only has the effects of dilating blood vessels and reducing heart load, but also has the effects of excreting sodium, diuresis, strengthening the heart, improving coronary circulation, protecting myocardium, improving microcirculation and the like.

Prostaglandin E1 alkyl esters are currently considered to be prodrugs of prostaglandin E1. For example, U.S. Patent No. 5,681,850 discloses prostaglandin E1 alkyl esters (C1-4) for the treatment of impotence. It is believed that prostaglandin E1 alkyl esters can be better absorbed through the skin by enhancing lipid solubility, and subsequently decomposed into prostaglandin E1 by hydrolase to take effect, so it is a prodrug; U.S. Patent No. 6,673,841 discloses a prostaglandin E1 alkyl ester (C1-5) external preparation, which contains prostaglandin E1 alkyl ester as a prodrug, an oily vehicle, a skin permeation enhancer and an anti-irritant agent.

JPH 08283160 discloses an emulsion composition for use as a therapeutic agent for peripheral circulatory incompetence such as Raynaud disease, burns, etc. The composition comprises: (A) a prostaglandin having inhibitory action on blood platelet aggregation and/or vasodilator action, preferably a compound of formula [1]: R¹ represents a hydrogen atom or a C₁₋₄ linear or branched alkyl group; R² represents R²¹ or (CH₂)ₙR²²; R²¹ is C₃₋₁₂ linear or branched alkyl, alkenyl or alkynyl group; R²² is a C₃₋₁₀ chloroalkyl; n is an integer of 0 to 2;. R³ and R⁴ are each independently a hydroxyl group or -OC(=O)R⁵; R⁵ is a C₁₋₅ straight-chain or branched alkyl group; (B) 10-30 parts by weight of a higher paraffin-based carbon (preferably a solid paraffin and/or vaseline); (C) 2-10 parts by weight of a higher alcohol (usually cetyl alcohol, etc.), (D) 0.01-1 parts by weight of a fatty acid ester (preferably diisopropyl adipate); and (E) a surfactant (preferably non-ionic) and a water phase component.

US5219885 discloses prostaglandin E1 derivatives as pharmacologically active agents, and pharmaceutical compositions containing these compounds, especially for transcutaneous administration.

"Efficacy of Prostaglandin E1 and Polysaccharide Sulfate in the Treatment of Scleroderma" (Longhua Wang Zhengyuan , Chin. J. Dermatol., vol. 29, no. 4, 1996), discloses that Prostaglandin E1 injection and polysaccharide sulfate tablets were first used to treat 36 cases and 31 cases with scleroderma in 1993, respectively, which were compared with 30 cases treated with compound Danshen injection. Satisfactory efficacy was obtained.

WO 2012/139033 discloses methods and compositions for treating Raynaud's disease and Raynaud's phenomenon. Topical administration of a semisolid composition comprising a prostaglandin E1 compound provides the desired relief of the Raynaud's disease or Raynaud's phenomenon without the possible complications of systemic administration. The semisolid composition can be administered as needed, or in a regimen of several doses per day.

However, the inventors have unexpectedly found in the research that the prostaglandin E1 methyl ester itself has a strong biological activity, thus further finding its medical use related to dilation of blood vessels.

### Summary of Invention

The disclosure provides prostaglandin E1 methyl ester for use in a method of treating scleroderma by reducing the skin thickness and/or collagen deposition of an animal with scleroderma, wherein the prostaglandin E1 methyl ester has a structure represented by formula (I).

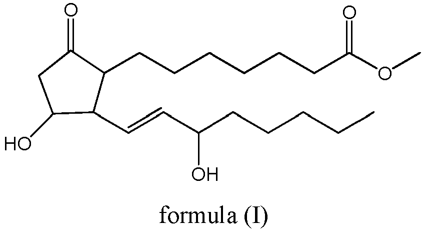

According to some specific embodiments of the disclosure, the animal is a mammal.

According to some specific embodiments of the disclosure, the animal is a human.

The inventors have unexpectedly found in the research that prostaglandin E1 methyl ester itself has a strong drug activity and can be directly affine with DP1 receptor. Activation of DP1 receptor can inhibit platelet aggregation and dilate blood vessels, rather than being a prodrug that needs to be hydrolyzed and delayed to take effect. In specific experimental examples, prostaglandin E1 methyl ester has shown better drug activity and therapeutic effect than prostaglandin E1, and a tissue distribution test has also shown that prostaglandin E1 methyl ester is more easily distributed in skin tissues.

### Brief Description of the Drawings

Figure 1 is a graph showing the variation of the inhibition rate with incubation time in Experimental Example 2;
Figure 2 is a graph showing the effect of the concentration of prostaglandin E1 methyl ester on the diastolic efficacy of isolated rabbit blood vessels in Experimental Example 3.

### Detailed Description

The technical solutions of the disclosure will be described in detail below in conjunction with the drawings and examples, but the protection scope of the disclosure includes but is not limited to these.

### Example 1

Synthesis of prostaglandin E1 methyl ester of the disclosure (methyl [(1R,2R,3R)-3-hydroxy-2-(S,E)-3-hydroxyoct-1-enyl)-5-oxocyclopentyl]heptanoate)

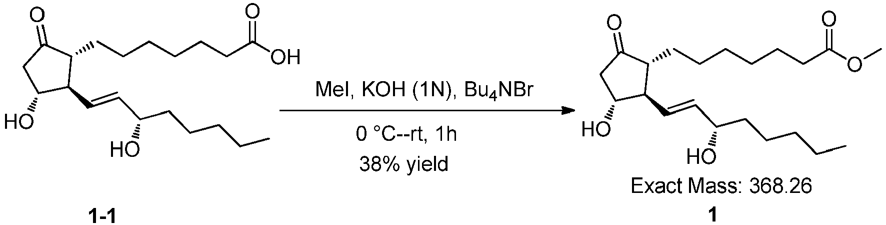

The starting material prostaglandin E1 (63 mg, 0.18 mmol) was added to a three-necked flask, and then a prepared 1M dry THF/Et₂O solution was added and stirred to dissolve. Under ice bath conditions, MeI (26 mg, 1M) solution was slowly added dropwise to the reaction solution, and after the completion of dropwise addition, KOH (10 mg, 0.18 mmol) and Bu₄NBr (6 mg, 0.018 mmol) were added. After the reaction solution was stirred for 1 h, it was heated to room temperature and monitored by TLC until the end of the reaction. The reaction was quenched by adding 20 ml of water. It was extracted with EtOAc (10 mL×3), and the organic phases were combined, dried over anhydrous Na₂SO₄ and filtered. The filtrate was concentrated under reduced pressure and purified by column chromatography (eluent n-hexane/EA= 1/1) to obtain a white solid product (24.8 mg, 38% yield).

LCMS (MS Found: 391.3 [M+Na]⁺. ¹HNMR (400MHZ, DMSO) (ppm): 5.46(s, 2H), 5.01(s, 1H), 4.57(s, 1H), 3.88(s, 2H), 3.57(s, 3H), 1.9-2.3(m, 5H)1.2-1.48(m, 19H), 0.85(s, 3H).

### Example 2

### Synthesis of Compound 2: (ethyl [(1R,2R,3R)-3-hydroxy-2-(S,E)-3-hydroxyoct-1-enyl)-5-oxocyclopentyl]heptanoate)

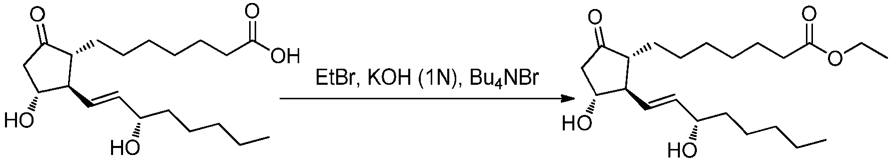

The starting material prostaglandin E1 (63 mg, 0.18 mmol) was added to a three-necked flask, and then a prepared 1M dry THF/Et₂O solution was added and stirred to dissolve. Under ice bath conditions, EtBr (20 mg, 1M) solution was slowly added dropwise to the reaction solution, and after the completion of dropwise addition, KOH (10 mg, 0.18 mmol) and Bu₄NBr (6 mg, 0.018 mmol) were added. After the reaction solution was stirred for 1 h, it was heated to room temperature and monitored by TLC until the end of the reaction. The reaction was quenched by adding 20 ml of water. It was extracted with EtOAc (10 mL×3), and the organic phases were combined, dried over anhydrous Na₂SO₄ and filtered. The filtrate was concentrated under reduced pressure and purified by column chromatography (eluent n-hexane/EA = 1/1) to obtain a white solid product (20.5mg, 29.8% yield).

LCMS (MS Found: 405 [M+Na]⁺. ¹HNMR (400MHZ, DMSO) (ppm): 5.46 (s, 2H), 5.01 (s, 1H), 4.57 (s, 1H), 3.88 (s, 2H), 3.57 (s, 3H), 1.9-2.3 (m, 7H) 1.2-1.48 (m, 19H), 0.85 (s, 3H).

### Experimental Example 1 (Reference)

Affinity test of DP receptor target in vitro.

The radioligand ([3H] Prostaglandin D2 (PGD2)) competitive receptor binding assay was used to evaluate the affinity of the test compound with the DP receptor. The results are shown in Table 1.

**Table 1**

| | Target | Species | Test Concentration (nM) | Inhibition rate |
|---|---|---|---|---|
| Prostaglandin E1 methyl ester | hDP1 | human | 100 | 78% |

The results show that Prostaglandin E1 methyl ester has a higher affinity with the DP receptor.

### Experimental Example 2 (Reference)

The effect of the prostaglandin E1 methyl ester of the disclosure in the anti-platelet aggregation test in vitro.

When healthy adult SD rats were anesthetized by intraperitoneal injection of 10% chloral hydrate, fresh whole blood was collected from the abdominal aorta and added to a centrifuge tube anticoagulated with 3.8% sodium citrate solution, and centrifuged at 900 rpm for 10 minutes to remove the upper platelet-rich plasma (PRP) for use. The tube having PRP removed was further centrifuged at 4000 rpm for 10 minutes, and the upper clarified plasma (PPP) was removed for use. In the experiment, Techlink model LBY-NJ4 4-channel platelet aggregator was used to determine the anticoagulant efficacy of each compound.

Into a sample cup containing 300µL of PRP, 2µL of 100µM prostaglandin E1, prostaglandin E1 methyl ester of example 1, Compound 2 of Example 2 and methanol (solvent) were first added. After incubated for different periods (0, 1, 2, 4, 7, 10, 15min), 20µL of aggregation inducer 180µM ADP solution was added. The aggregation rate of each sample was measured, and the inhibitory rate of the compound on ADP-induced platelet aggregation was calculated. Inhibition rate% = (solvent aggregation rate - compound aggregation rate)/solvent aggregation rate × 100%

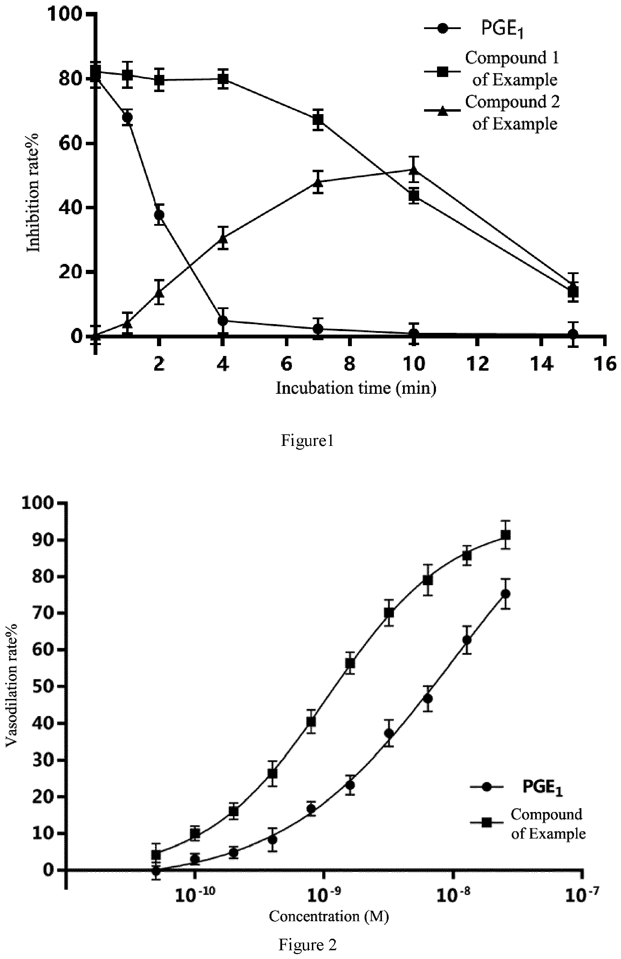

From the results (Figure 1), it can be seen that the additions of prostaglandin E1 and prostaglandin E1 methyl ester of the Example to PRP take effect immediately, and the inhibition rates were equivalent. With prolonged incubation time, the anticoagulant efficacy of the compound of the Example slowly decreased, and the inhibition rate is still 43.66% after 10 minutes, but the inhibition rate of prostaglandin E1 is only 4.93% after 4 minutes of incubation. Compound 2 of Example 2 does not take effect immediately after the addition, and the efficacy gradually increased over time, and reaches the maximum inhibition rate of 51.86% after 10 minutes of incubation. Therefore, the prostaglandin E1 methyl ester of Example 1 is an active non-prodrug compound, and its anticoagulant effect is 2 times longer than that of prostaglandin E1, and Compound 2 of Example 2 is a typical prodrug compound.

### Experimental Example 3 (Reference)

The effect of the prostaglandin E1 methyl ester of the disclosure in the vasodilation test in vitro

In the experiment, rabbits were selected to prepare isolated aortic ring specimens: New Zealand white rabbits, male, weighing (2.5±0.3) kg. The rabbit was stunned with a blunt instrument, fixed on the rabbit dissecting table, and the thoracic aorta was quickly separated, and placed in a petri dish filled with saturated Kerbs solution (containing NaCl 6.9 g, KCl 0.35 g, MgSO₄·7H₂O 0.29 g, KH₂PO₄ 0.16 g, NaHCO₃ 2.1 g, CaCl₂ 0.28 g, glucose 2 g per 1000 mL) at 37°C and continuously introduced with mixed gas (95% O₂, 5% CO₂). The remaining blood in the blood vessel was squeezed out, and the peripheral fat and connective tissue were carefully peeled off, and it was cut into 0.5 cm long arterial rings for use. Two stainless steel L-shaped hooks were used to pierce through the vascular lumen of the vascular ring, and the vascular ring was hung horizontally in a 20 mL bath tube, fixed at the bottom, and connected to a tension transducer with a thin steel wire at the top. The resting tension was first adjusted to 0.00 g, and after stabilization for 20 minutes, 3.00 g tension was applied, and the tension level was continuously adjusted to maintain it at about 3.00 g and stabilized for 2 h (replacing the Kerbs solution along the wall of the bath every 15 minutes).

BL-420S biological function experiment system (Chengdu Techman Technology) was used to record the variation of vascular ring tension. After the vascular ring contraction was stable, prostaglandin E1 and the compounds of the Examples were accumulatively added to successively increase the final mass concentration of prostaglandin E1 in the bath tube to 0.05, 0.1, 0.2, 0.4, 0.8, 1.6, 3.2, 6.4, 12.8, 25.6 nM, and the diastolic efficacies of the vascular ring were recorded.

The results (Figure 2) show that under the experimental conditions, the diastolic efficacy of the prostaglandin E1 methyl ester of Example 1 on isolated rabbit blood vessels (EC50=1.090 nM) is significantly stronger than that of prostaglandin E1 (EC50=9.767 nM).

### Experimental example 4 (Reference)

Frozen human umbilical vein endothelial cells (HUVEC) were resuscitated and placed in low-sugar DMEM medium with 10% (V/V) FBS, and 100U/L penicillin and 0.1g/L streptomycin were added. It was cultured artificially in an environment of 5% CO₂ and 95% air at a temperature of 37°C. A fresh medium was replaced every three days. When the cell density reached 70-80%, the digestion solution (0.25% trypsin/0.02EDTA) was used to harvest the cells. After the cells were resuspended, they were seeded in a 96-well plate with a density of 5×10³ per well.

After 1 day of culture, 1-100 nM of prostaglandin methyl ester was added and incubated for 24 hours for MTT detection. Compared with the cells in the unmedicated wells, the prostaglandin methyl ester has a significant effect of promoting the proliferation of vascular endothelial cells, which can promote angiogenesis and improve vascular function, and can be used clinically to treat microcirculation disorders.

### Experimental example 5

Therapeutic effect of prostaglandin E1 methyl ester in mouse scleroderma model

Scleroderma is a connective tissue disease characterized by fibrosis of the skin, blood vessels and internal organs, with a large number of autoantibodies, which destroy various cellular components, produced in the body. Its pathogenesis has three basic processes, namely fibrosis, inflammation and vascular dysfunction. The etiology and pathogenesis of the disease are still not fully understood, and there is no ideal treatment drug and method.

In this experiment, 56 Balb/c mice weighing 22-25g were randomly divided into 7 groups: normal group, model group, vehicle group, prostaglandin E1 high-dose group and prostaglandin E1 low-dose group, and prostaglandin E1 methyl ester high-dose group and prostaglandin E1 methyl ester low-dose group.

Model preparation: the clothing hair on the central area of the mice was shaved, the normal group was injected with 0.1ml PBS subcutaneously on the back, and the other groups were injected with 0.1ml of 0.2mg/ml bleomycin subcutaneously, once a day for three weeks.

Method of administration: Osmotic pumps (model 1004, ALZA Corporation, Canada) containing 100µl of drug in DMSO were used. The drug was released at a constant rate, and the drug release cycle was 28 days. Prostaglandin E1 high and low-dose groups were loaded with 28µg and 14µg of drugs respectively; prostaglandin E1 methyl ester high and low-dose groups were also loaded with 28µg and 14µg of drugs respectively; solvent group was not loaded with drugs. The osmotic pump was buried in the mouse's abdominal cavity and the inferior vena cava was intubated for administration. The administration was started at the same time as the model was created.

Index detection: After the administration, the animals were sacrificed, and skin and lung sections of the injection site were made, and HE stained. The histological changes were observed, and the skin (dermis) thickness was measured; and the photoelectric colorimetry was used to determine the content of hydroxyproline and protein in the skin to infer the content of collagen.

The results showed that the dermal layer at the injection site of the model group was significantly thickened, collagen fibers were thickened with number increased, and the fibrous space was narrowed with atrophy of hair follicles, the blood vessel wall was thickened, the lumen was narrowed, and inflammatory cell infiltration was accompanied. The local skin of the mice in each administration group was thickened to different degrees, but they were all thinner than the model group. The collagen fibers were arranged loosely and the hair follicles had less inflammatory cell infiltration. The skin thickness of the prostaglandin E1 group and the prostaglandin E1 methyl ester group were dose dependent. The high-dose group of the same drug was better than the low-dose group, and the prostaglandin E1 methyl ester group was significantly better than the prostaglandin E1 group. In lung tissue, there was thickening of alveolar septum with a large number of monocyte infiltration. There was fibroblast proliferation in the gap, and the wall of small blood vessels was thickened. Compared with the model group, the thickening of the alveolar septum in each dose group was reduced, and the infiltration of inflammatory cells was slightly relieved. The prostaglandin E1 methyl ester group was slightly better than the prostaglandin E1 group.

Excessive deposition of collagen in the skin and corresponding visceral tissues plays an important role in the development of scleroderma. The results show that the skin collagen content of the model group is significantly increased, and the average collagen content of each administration group was lower than that of the model group. However, there is no significant difference between the two dose groups of prostaglandin E1, and the protein content of the two dose groups of prostaglandin E1 methyl ester is significantly lower than that of the model group and the two dose groups of prostaglandin E1. The skin thickness and skin collagen content of mice in each group are shown in Table 2 below.

**Table 2 Comnarison of skin thickness and skin collagen content of mice in each group**

| Groups | Dosage | Skin thickness (µm) | Collagen content (mg/L) |
|---|---|---|---|
| Normal | / | 18.29±2.08 | 325.31±113.87 |
| Model | / | 42.71±2.56 | 1062.33±253.31 |
| Solvent | / | 41.35±1.99 | 1018.19±345.15 |
| prostaglandin E1 high-dose | 1ug/day | 29.12±2.46* | 879.66±128.92 |
| prostaglandin E1 low-dose | 0.5 ug/day | 32.59±3.03* | 947.99±214.86 |
| prostaglandin E1 methyl ester high-dose | 1ug/day | 21.35±2.11*# | 467.52±141.08*# |
| prostaglandin E1 methyl ester low-dose | 0.5ug/day | 24.66±2.54*# | 588.62±127.41*# |

| | | | |
|---|---|---|---|
| *Compared with the model group, p<0.05; #Compared with the prostaglandin E1 high-dose group, p<0.05 | | | |

The above results indicate that the prostaglandin E1 methyl ester has an excellent improvement effect on the mouse scleroderma model and is better than that of prostaglandin E1.

### Experimental example 6 (Reference)

### Distribution of prostaglandin E1 methyl ester in rat skin tissue

In this experiment, 12 male SD rats weighing 250-280g were randomly divided into two groups (n=6): prostaglandin E1 group and prostaglandin E1 methyl ester group. Osmotic pumps (model 1003D, ALZA Corporation, Canada) containing 100µl of drug in DMSO were used. The drug was released at a constant rate, and the drug release cycle was 3 days. They were loaded with 200µg of [³H] prostaglandin E1 and [³H] prostaglandin E1 methyl ester respectively. The osmotic pump was buried in the rat's abdominal cavity and the inferior vena cava was intubated for administration. After the administration, the animals were sacrificed, skin tissues were taken (the same position for each animal), weighed, and the total radiation value was measured. The results are shown in Table 3.

**Table 3**

| Groups | Skin tissue concentration ng (radiation equivalent)/g |
|---|---|
| prostaglandin E1 group | 45.1±7.5 |
| prostaglandin E1 methyl ester group | 139.3±18.6* |

| | |
|---|---|
| *: P<0.05 | |

The results show that under the same dosage, the skin tissue concentration of prostaglandin E1 methyl ester is significantly higher than that of prostaglandin E1.

## Claims

1. Prostaglandin E1 methyl ester for use in a method of treating scleroderma by reducing the skin thickness and/or collagen deposition of an animal with scleroderma, wherein the prostaglandin E1 methyl ester has a structure represented by formula (I).

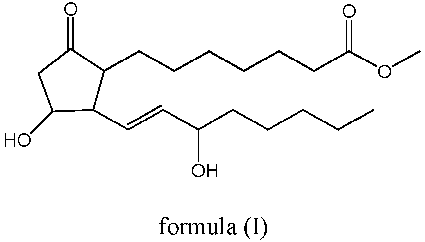

2. The prostaglandin E1 methyl ester for use according to claim 1, wherein the animal is a mammal.

3. The prostaglandin E1 methyl ester for use according to claim 1, wherein the animal is a human.

## Patentansprüche

1. Prostaglandin E1-Methylester zur Verwendung in einem Verfahren zur Behandlung von Dermatosklerose durch Verringern der Hautdicke und/oder Kollagenablagerung eines Tiers mit Dermatosklerose, wobei das Prostaglandin E1-Methylester eine durch Formel (I) dargestellte Struktur aufweist.

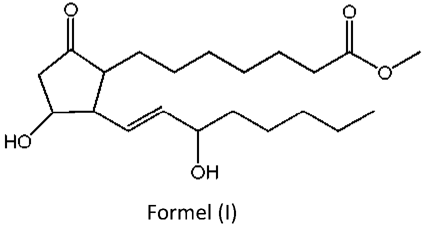

2. Prostaglandin E1-Methylester zur Verwendung nach Anspruch 1, wobei es sich bei dem Tier um ein Säugetier handelt.

3. Prostaglandin E1-Methylester zur Verwendung nach Anspruch 1, wobei es sich bei dem Tier um einen Menschen handelt.

## Revendications

1. Ester méthylique de prostaglandine E1 pour utilisation dans un procédé de traitement de la sclérodermie par réduction de l'épaisseur cutanée et/ou du dépôt de collagène d'un animal atteint de sclérodermie, dans lequel l'ester méthylique de prostaglandine E1 présente une structure représentée par la formule (I).

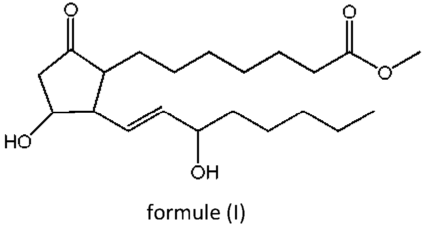

2. Ester méthylique de prostaglandine E1 pour utilisation selon la revendication 1, dans lequel l'animal est un mammifère.

3. Ester méthylique de prostaglandine E1 pour utilisation selon la revendication 1, dans lequel l'animal est un être humain.
